# EUROPEAN PATENT APPLICATION

(11) **EP 1 248 213 A1**
(43) Date of publication of application: **09.10.2002**
(21) Application number: 00979097.3
(22) Date of filing: 05.12.2000
(51) Int. Cl.: G06F 17/60

(54) **HEALTH ADVISING METHOD AND HEALTH ADVISING SYSTEM**

(30) Priority: 10.12.1999 JP 352399; 07.08.2000 JP 2000239218
(71) Applicant: Health Wave Japan Inc., Tokyo 101-0032 (JP)
(72) Inventor: WAKABAYASI, Hidetake, c/o Health Wave Japan Inc., Chiyoda-ku, Tokyo 101-0032 (JP)
(74) Representative: Pfenning, Meinig & Partner
(86) International application number: JP0008602
(87) International publication number: WO01043003

(57) **Abstract**

The present invention is to provide a health advising method and a health advising system which can prepare at any time information required for a person who wants to be advised about his or her health, and which enables a person who gives advice to sufficiently advise to many people.

An adviser receives application from a user to the effect that the user wants to be advised about health, and then sends a question sheet about life, a kit for storing blood, and a blood-collecting needle to the user by mail. When the question sheet about life in which answers of the user are filled, the kit into which blood of the user has been dropped, and the blood-collecting needle are sent back, the adviser inspects the blood in the kit, and then inputs results of the inspection and the answers to the questions into a morbid state analysis expert system. The morbid state analysis expert system creates advice for the user on the basis of the inputted data and knowledge accumulated in a database. The adviser reports the created advice to the user.

## Description

### Technical Field

The present invention relates to a method and a system which give a user advice about how to improve user's health according to information about user's health.

### Background Art

In recent years, life-style related diseases are increasing, which presents a problem. The life-style related diseases are a group of diseases, onset and progress of which are considered to be influenced by living habit such as eating habits, exercise habit, rest, smoking, and drinking. The life-style related diseases include cancer, diabetes, and cerebral apoplexy, for example. It is not easy to treat these life-style related diseases after onset of a disease. For this reason, primary prevention of life-style related diseases is important. To be more specific, it is important to prevent onset of a disease beforehand by managing health by ourselves.

It is said that carrying out the primary prevention and improving living habit enable us to avoid life-style related diseases. However, even if we try to improve living habit in a state in which subjective symptoms do not develop, in most cases, we cannot know how to improve living habit specifically using personal knowledge. Therefore, trying to carry out the primary prevention after having appropriate advice by an expert such as a doctor has the effect of avoiding life-style related diseases and of delaying progress thereof.

Nevertheless, when we try to be advised on our health from the expert, there are problems as follows. Firstly, a problem to be considered is that it is difficult for a person who wants to be advised to have time it takes to be advised. Secondly, another problem to be considered is that because there are many people whom an expert should advise, the expert cannot find sufficient time for advising each person, which may result in inability to keep the quality of advice. Because most of target persons to be advised are healthy, the expert must give advice to a great number of people. However, there is provided no system suitable for giving advice for such many people.

An object of the present invention is to provide a health advising method and a health advising system which can prepare at any time information required for a person who wants to be advised about his or her health, and which enables a person who gives advice to sufficiently advise to many people.

### Disclosure of Invention

A health advising method according to the present invention is characterized by comprising the steps of:
in response to application from a user, sending a medium for storing information about health to the user;
receiving the medium in which the user has stored information about health;
extracting the information about user's health stored in the medium to input said information in a morbid state analysis expert system;
creating advice about user's health by the morbid state analysis expert system; and
reporting the advice about user's health created by the morbid state analysis expert system to the user.

In addition, a health advising system according to the present invention is characterized by comprising:
a morbid state analysis expert system that inputs information about health extracted from a medium for storing information about user's health, and that creates advice about user's health.

In addition, the medium for storing information about health may comprise a question sheet about life in which questions are described, and in which answers to the questions are written by the user; a blood-collecting tool used for dropping blood of the user; and a kit for storing the dropped blood; and the morbid state analysis expert system may create advice about user's health on the basis of the answers to the questions written in the question sheet about life, and on the basis of components of the blood stored in the kit. If the morbid state analysis expert system has such a configuration, when giving advice about health, the medium for storing information about health can be sent by mail simply; in addition, a person who tries to be advised about health can fill in the question sheet about life and collect blood whenever convenient to the person.

The medium for storing information about health may comprise a blood-collecting tool used for dropping blood of the user; and a kit for storing the dropped blood; and the morbid state analysis expert system may receive answers to given questions via a communication network, and creates advice about user's health on the basis of components of the blood stored in the kit and the answers. If the morbid state analysis expert system has such a configuration, a person who tries to be advised about health can transmit the answers to the questions and collect blood whenever convenient to the person.

For example, the kit for storing blood may have a separating agent for separating blood into a blood clot and serum, said kit being mounted on a centrifugal separator by a user, said kit storing the blood clot and the serum which are separated by the separating agent and by centrifuging the blood; and the morbid state analysis expert system creates advice about user's health on the basis of components of the separated serum. According to such a configuration, because the blood is separated into the blood clot and the serum by the user, a component discharged by hemolysis does not mix with the serum, which enables broad inspection using the serum.

In addition, for example, the kit for storing blood may have a preservative for preventing hemolysis, and store blood together with the preservative; and the morbid state analysis expert system may create advice about user's health on the basis of components of the blood stored together with the preservative. According to such a configuration, because the blood is stored together with the preservative, the blood does not hemolyze, which enables broad inspection.

In a health advising method according to the present invention, preferably the medium for storing information about health may comprise a blood-collecting tool used for dropping blood of the user; and a kit for storing the dropped blood; and in a step in which, in response to application from a user, a medium for storing information about health may be sent to the user, a centrifugal separator for separating the blood in the kit into a blood clot and serum is sent together with the medium to the user who makes application for the first time. Separating the blood into the blood clot and the serum by centrifuging the blood immediately after collecting the blood enables the user to prevent a component discharged by hemolysis from mixing with the serum. In addition, the centrifugal separator can be used repeatedly. Therefore, if the centrifugal separator is sent to the user who makes application for the first time to the effect that the user wants to be advised about health, it is not necessary to send the centrifugal separator again, which can reduce costs required for advice.

### Brief Description of Drawings

Fig. 1 is an explanatory diagram illustrating how to give and receive information between a user and an adviser;
Fig. 2 is an explanatory diagram illustrating an example of a kit for storing blood;
Fig. 3 is an explanatory diagram illustrating an example of a funnel;
Fig. 4 is an explanatory diagram illustrating an example of a simple centrifugal blood separator;
Fig. 5 is an explanatory diagram illustrating a blood clot and serum which are separated;
Fig. 6 is an explanatory diagram illustrating a flow of information after receiving a medium in which an adviser stores information about health;
Fig. 7 is an explanatory diagram illustrating blood components to be inspected and an example of inspection purposes;
Fig. 8 is an explanatory diagram illustrating an example of a medical advice report;
Fig. 9 is an explanatory diagram illustrating an example of a report on advice about life-style related diseases;
Fig. 10 is an explanatory diagram illustrating an example of a report on advice about nutrition;
Fig. 11 is an explanatory diagram illustrating an example of a report on advice about exercises;
Fig. 12 is a block diagram illustrating an example of a configuration of a morbid state analysis expert system that is applied to the present invention; and
Fig. 13 is a flowchart illustrating an example of operation until the morbid state analysis expert system determines contents to be reported.

### Best Modes for Carrying out the Invention

Embodiments of the present invention will be described hereinafter with reference to drawings.

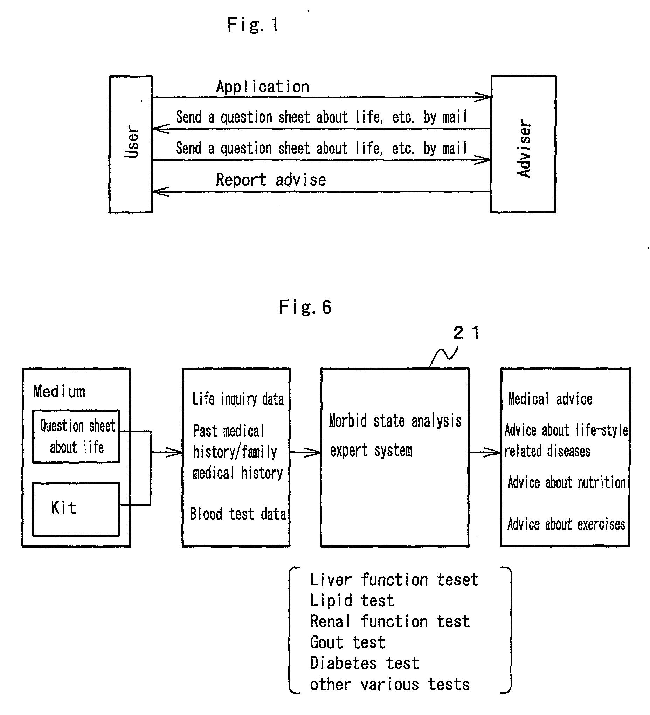

Fig. 1 is an explanatory diagram illustrating how information is given and received between an adviser and a user who is advised about his or her health. In the first place, the adviser receives application from the user to the effect that the user wants to be advised about health. The adviser may directly receive application from the user, or may also receive application through an agency. The adviser who has received the application sends a question sheet about life, a kit for storing blood, a blood-collecting needle, and a funnel to the user by mail. In addition, the adviser also sends by mail a simple centrifugal blood separator, which is used for separating blood in the kit into a blood clot and serum, to the user who made application for the first time to the effect that the user wants to be advised. When sending the user the kit, and the like, they may also be sent by a home delivery service instead of by mail.

The question sheet about life is a sheet in which doctor's questions about life of a user, and the like, are described; more specifically, doctor's questions about life such as past medical history, family history (family medical history), meals, and sleep are described in the question sheet. The user fills answers to the questions in the question sheet. A format of the answers is, for example, a mark sheet format. The kit stores blood which the user has collected. Answers to the questions and components included in the blood are treated as information showing a condition of user's health. To be more specific, the question sheet about life and the kit are used as media for saving the information showing the condition of the user's health. In addition, a blood-collecting tool (hereinafter referred to as blood-collecting needle) is, for example, a lancet, which is used when the user collects blood. The user pricks his/her finger, or the like, with the blood-collecting needle to drop blood flowing from the finger, or the like, into the kit.

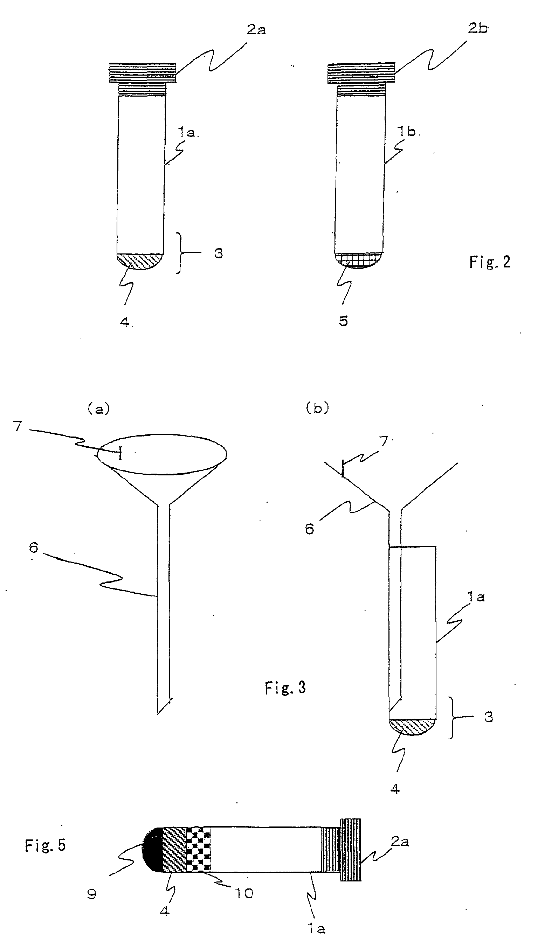

Fig. 2 is an explanatory diagram illustrating an example of the kit for storing blood. The kit comprises blood collecting tubes 1a, 1b. Caps 2a, 2b are attached to the blood collecting tubes 1a, 1b respectively. An area near the bottom of the blood collecting tube 1a is coated with coagulation accelerating agent 3 such as silica and thrombin. Additionally, at the bottom of the blood collecting tube 1a, a separating agent 4 for separating blood into a blood clot and serum is placed. On the other hand, at the bottom of the blood collecting tube 1b, preservative 5 for preventing hemolysis is placed. Each of the blood collecting tubes 1a, 1b has a length of about 3 cm.

Fig. 3 (a) illustrates a shape of a funnel as an example. A funnel 6 has a guide bar 7, which is used for guiding blood into the blood collecting tubes 1a, 1b with ease. In addition, the funnel 6 is coated with silicon. When dropping blood into the blood collecting tube 1a, the cap 2a is uncapped. Then, as shown in Fig. 3 (b), the funnel 6 is inserted into the blood collecting tube 1a to drop blood of about 150 through 200 µ liter (equivalent to three drops) from the guide bar 7. It is desirable that a tubular portion of the funnel 6 be long enough to reach an area near the separating agent 4 so that the blood does not adhere to an upper part of the blood collecting tube 1a. Blood is also dropped into the blood collecting tube 1b in a like manner. When the blood collecting tube 1a is rotated using a centrifugal separator, the separating agent 4 and the blood clot are reversed in the blood collecting tube 1a, and thereby the blood clot and the serum are completely separated. Blood in the blood collecting tube 1b is stored using the preservative 5 without hemolysis.

Fig. 4 is an explanatory diagram illustrating an example of a simple centrifugal blood separator. The blood collecting tube 1a, into which blood is put, is mounted on a simple centrifugal blood separator 8 while the cap 2a is in a closed state. Then, the blood collecting tube 1a is centrifuged so as to reverse the separating agent 4 and the blood clot, and thereby the blood clot and the serum are completely separated. The simple centrifugal blood separator 8 has a size that is capable of mounting one blood collecting tube 1a. The blood collecting tube 1a is mounted so that its central part corresponds to a center of a disk. Rotating the disk at the number of revolutions of 5000 through 20000 rpm enables separation of serum from a very small amount of blood ranging from 150 to 200 µ liter (equivalent to three drops) within two minutes. If a commercial small-size motor and a dry battery (size AA battery of DC 3V) are used, the centrifugal separator can be driven at this number of revolutions. The user mounts the blood collecting tube 1a on the simple centrifugal blood separator 8, and then rotates the blood collecting tube 1a to separate the blood into the blood clot 9 and the serum 10 as shown in Fig. 5. The blood clot 9 and the serum 10, which are separated by the separating agent 4, do not mix even if the blood collecting tube 1a is shaken.

As described below, the kit into which blood has been dropped is sent to the adviser by mail, or the like. In the process of sending by mail, or the like, the blood hemolyzes due to shock if preservative is not included. To be more specific, a blood cell breaks. If blood hemolyzes, inspection cannot be performed for the most part. However, if the serum and the blood clot are separated as described above, a component discharged by hemolysis does not mix with serum, which enables broad inspection using serum. It is to be noted that it is not necessary to rotate the blood collecting tube 1b by the centrifugal separator.

Next, as shown in Fig. 1, the user sends the adviser the following by mail: the question sheet about life in which answers have been filled; the kit into which blood has been dropped; the blood-collecting needle; and the funnel. The kits, and the like, may also be sent by home delivery service, or the like. The adviser, who has received the kit, and the like, extracts blood in the kit to inspect its components. To be more specific, components of the serum in the blood collecting tube 1a and of the blood which is not hemolyzed in the blood collecting tube 1b are inspected. The adviser may also consign the inspection to a sanitary inspection center. Alternatively, the adviser himself/herself with the qualification as a clinical inspecting engineer may also perform the inspection.

After that, results of the inspection and answers of the question sheet about life are inputted into a morbid state analysis expert system. The morbid state analysis expert system creates appropriate advice for the user using the inputted data. In addition, as described below, the morbid state analysis expert system comprises a database. The morbid state analysis expert system manages data of the created advice using the database. When creating the advice, if there is past advice data relating to the same user, the past data is also shown in the created advice.

The morbid state analysis expert system prints out the created advice as a report. However, it does not print out the report, but another terminal equipment which receives contents of the advice as electronic data from the morbid state analysis expert system may also print out the report.

The adviser sends this report to the user. The adviser may also send the report to the user directly, or may also send it to the user through an agency. It is to be noted that the adviser may also transmit the advice as electronic data to terminal equipment of the agency so that the agency can print out the report to send it to the user.

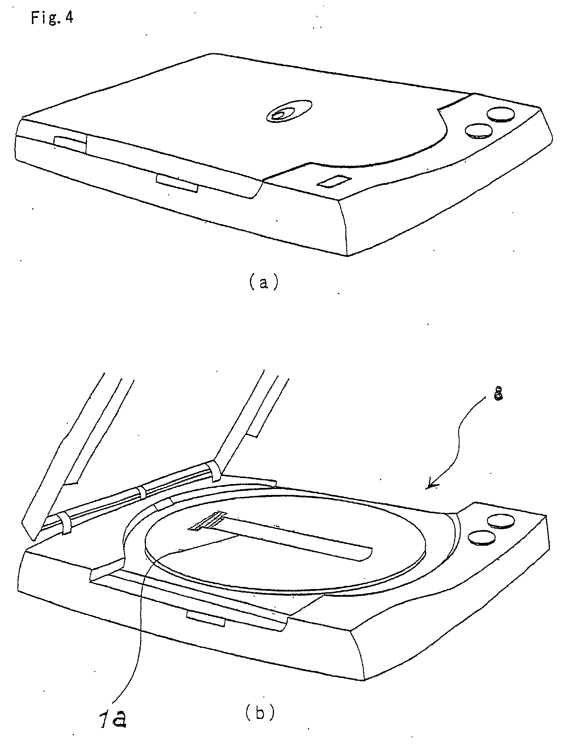

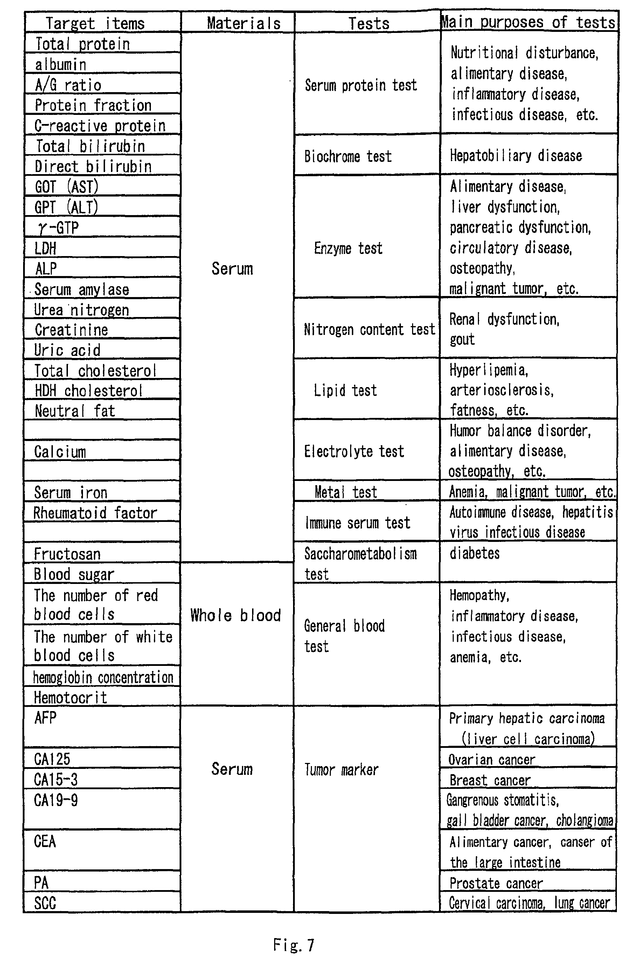

Fig. 6 is an explanatory diagram illustrating a flow of information until advice about health is created after the adviser receives the question sheet about life and the kit. When the adviser receives the question sheet about life and the kit, the adviser extracts information about user's health included in them. For the question sheet about life, life inquiry data, the past medical history, the family medical history, and the like obtained from the answers correspond to information about health. For the kit, blood test data obtained from blood test in the kit corresponds to information about health. The blood test data means inspection data such as neutral fat, uric acid, urea nitrogen, creatinine, a red blood cell, and a white blood cell, for example. Fig. 7 exemplifies blood components to be inspected and purposes of the inspection. It is to be noted that using the serum in the blood collecting tube 1a and the blood in the blood collecting tube 1b enables inspection for the most part. In addition, purposes of inspection are not limited to those shown in Fig. 7.

The information about health is inputted into the morbid state analysis expert system 21. The expert system 21 performs various kinds of tests, for example, such as a liver function test, a lipid test, a renal function test, a gout test, and a diabetes test on the basis of the inputted information about health, and then creates various kinds of inspection results and advice about user's health to output them. Contents of the advice include the following: medical advice that is advice about disease expected in the future and a current level of health; advice about life-style related diseases that is advice about prevention of disease expected in the future and improvement of living conditions for health maintaining; advice about nutrition that is advice about eating habits; and advice about exercises that is advice about an exercise menu. The adviser sends the advice report to the user. In addition, contents of the advice to the user are accumulated in the database. When advising the same user again, the contents of the advice are used to indicate how the condition of user's health has changed.

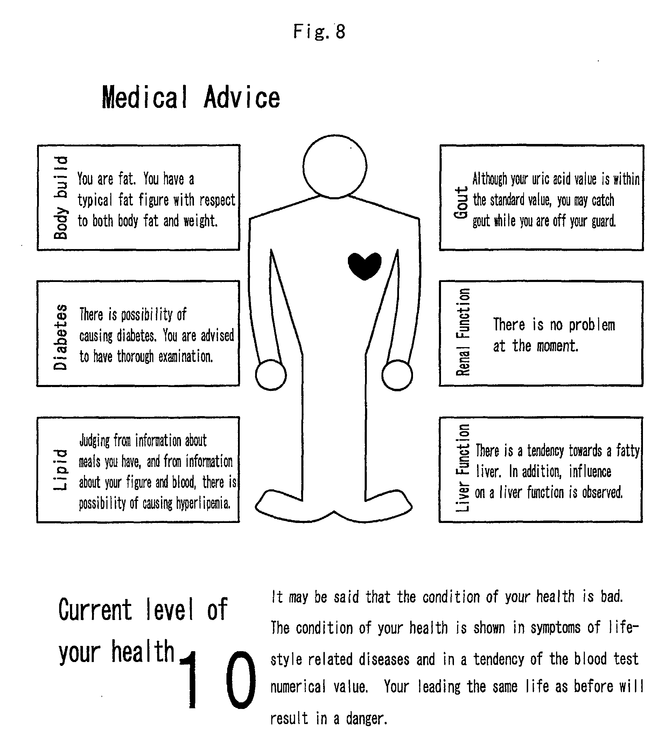

Fig. 8 is an explanatory diagram illustrating an example of a medical advice report. In the medical advice report, for example, an overall level of health, and result of each inspection such as the liver function test, which has been performed by the morbid state analysis expert system, are described. Moreover, specific numerical values obtained as a result of blood component inspection, and other information, may also be described.

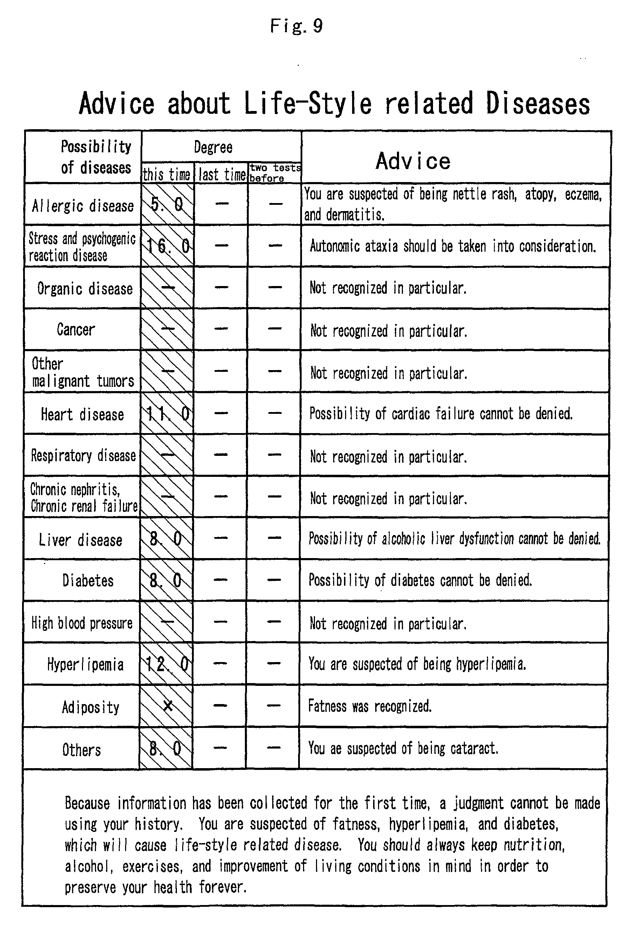

Fig. 9 is an explanatory diagram illustrating an example of a report on advice about life-style related diseases. In the report on advice about life-style related diseases, for example, a state of the user's body and advice about improvement of living conditions are described. In addition, items judged from the answers of the doctor's questions, and other information, may also be described.

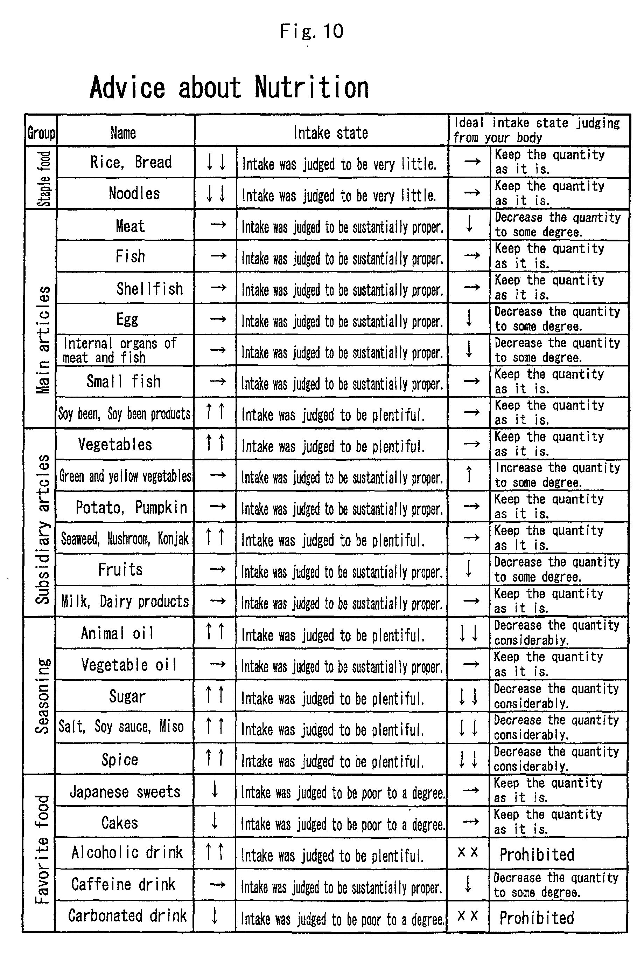

Fig. 10 is an explanatory diagram illustrating an example of a report on advice about nutrition. In the report on advice about nutrition, ideal food intake states, and the like, are described. In addition, life-style related diseases which will appear if the user continues the same eating habits as before, and other information, may also be described.

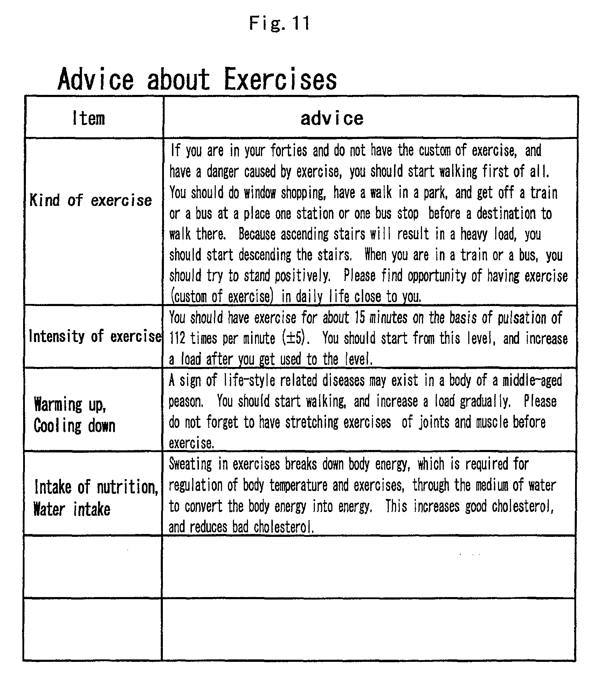

Fig. 11 is an explanatory diagram illustrating an example of a report on advice about exercises. In the report on advice about exercises, advice about exercises which are suitable for the user, and other information, are described. In addition, life-style related diseases which will appear if the user does not take advised exercises, and other information, may also be described.

If past data of the same user is managed in the database, as shown in Fig. 9, for example, columns such as "Last time" and "Two tests before" are provided in order to show change in state up to the present time. In addition, the advice report created in the past may also be printed out again to report it to the user together with a newly created report.

The morbid state analysis expert system may also create a report on a part of the advice described above. Alternatively, the morbid state analysis expert system may also create advice except the above.

Inspection for the most part in addition to life-style related diseases becomes possible from components of blood. For example, allergy and sexually transmitted diseases can be inspected. The expert system may create advice about various kinds of diseases such as allergy and sexually transmitted diseases, except life-style related diseases, based on data of blood component inspection to report its result to the user.

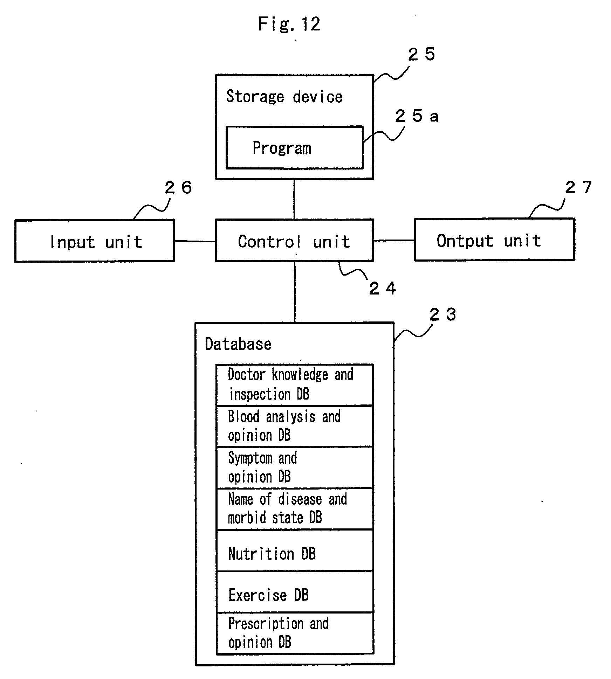

Fig. 12 is a block diagram illustrating an example of a configuration of the morbid state analysis expert system 21. The control unit 24 controls the morbid state analysis expert system 21 according to a program 25a stored in a storage device 25. An input unit 26 comprises an optical mark reader and a keyboard. The input unit 26 is used to input results of a blood test and answers by the user in a mark sheet format or the like. The program 25a is a program that has a function of creating advice based on data inputted from the input unit 26 and data in the database 23. An output unit 27 comprises a printer, and prints an advice report. The output unit 27 may also be configured as an interface that transmits the created advice as electronic data to another terminal equipment through a communication line. In this case, the terminal equipment that will receive the electronic data prints a report.

The database 23 comprises the following: a database in which knowledge possessed by a doctor is accumulated; a database in which results of a blood test and an opinion about the test results are accumulated; a database in which various kinds of symptoms and an opinion about each symptom are accumulated; a database relating to names of diseases and morbid states; a database relating to advice about nutrition; a database relating to advice about exercises; and a database in which advice and an opinion about a user are accumulated.

In the database 23, a lot of knowledge is accumulated in the following formats: if A1, it is X1; if A2, it is not X2; if A1 and A3, it is X3; and the like. In this case, A1, A2, A3, X1, X2, and X3 are data stored in the database 23. Data such as A1, A2, and A3 are inputted into the control unit 24. Then, contents to be advised are determined based on knowledge accumulated in the database 23 and inputted data.

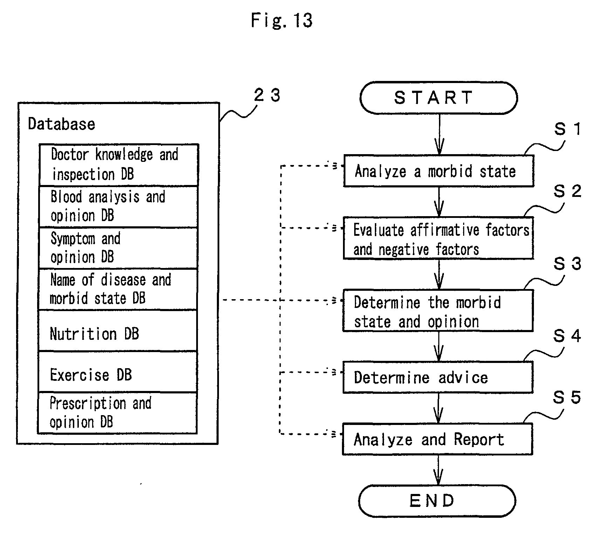

Fig. 13 is a flowchart illustrating an example of operation taken until the morbid state analysis expert system 21 determines contents to be reported. Each step of the flowchart shown in Fig. 13 is executed based on the knowledge of the database 23. The control unit 24 analyzes a morbid state of the user using blood test data, and the like (step S1). To be more specific, the control unit 24 carries out an analysis to check whether or not the user is healthy, or what kind of disease the user may have. Subsequently, the control unit 24 evaluates both of a factor as grounds for indicating a morbid state of the user (affirmative factor) and a factor showing that the user does not have the morbid state (negative factor) (step S12). For example, if it is judged that there is possibility of cancer in a step S1 as a result of the analysis, both of data showing that it is cancer and data showing that it is not cancer are evaluated, and thereby data to which priority should be given is determined. Next, the control unit 24 determines a morbid state of the user, and an opinion, judging from the analyzed morbid state, and judging from the evaluation result of the affirmative and negative factors of the morbid state (step S3).

After determining the morbid state and the opinion, the control unit 24 determines advice about the morbid state of the user (step S4), and then creates various advice reports (step S5).

In the conventional morbid state analysis expert system, a morbid state is determined only by an affirmative factor. To the contrary, the morbid state analysis expert system 21 applied to the present invention determines a morbid state of a user considering not only an affirmative factor as grounds for indicating the morbid state of the user but also a negative factor showing that the user does not have the morbid state. Thus, according to the present invention, a morbid state can be determined with higher accuracy, with the result that advice can be provided to the user more accurately.

The adviser reports contents of the advice to the user. Acting on the advice at an initial stage prevents the user from suffering from life-style related diseases. In addition, if the user has ever been advised on health, past data are also provided. Therefore, it is possible to check change in condition of health over a long period. Moreover, the user can be advised about allergy and sexually transmitted disease as well as life-style related diseases.

According to such a health advising method, the user can be advised about health without visiting the adviser. Further, the user can write answers in a question sheet about life and collect blood at user's convenience. This solves the problem that it is difficult for the user to make time it takes to have advice.

In addition, according to the health advising method of the present invention, advice is created by the morbid state analysis expert system 21. Therefore, the problem that the adviser cannot spend sufficient time for advising a lot of people is also solved. Therefore, the quality of advice can be kept even when advice is given to a lot of people.

Additionally, the blood in the blood collecting tube 1a is sent to the adviser after the user subjects the blood to centrifugation to separate the blood into a blood clot and serum. The blood in the blood collecting tube 1b is sent to the adviser together with the preservative 5 for preventing hemolysis. This avoids inability to inspect the blood due to hemolysis. Accordingly, as exemplified in Fig. 7, the adviser can give broad advice judging from inspection data relating to many components.

The simple centrifugal blood separator 8 is kept on the user side, and is used again when the user is advised next time. Therefore, if from the user whom the adviser has given advice, the adviser receives application to the effect that the user wants to be advised again, sending a question sheet about life, a kit (blood collecting tubes 1a, 1b), a blood-collecting needle, and a funnel, without sending a centrifugal separator, will suffice. As a result, it becomes possible to reduce costs required when the adviser gives advice to the same user multiple times.

Next, an embodiment in which a question sheet about life is not used will be described. In this embodiment, an adviser has a server connected to the Internet; and a user has terminal equipment connected to the Internet. The server and the morbid state analysis expert system 21 are connected to each other via a communication network such as LAN in order to transmit and receive information. In addition, the server holds WEB page screen information that presents the same items as doctor's questions described in the question sheet about life in the above-mentioned embodiment. The WEB page displays columns for questions about user's life and columns for answers. On the other hand, terminal equipment of the user holds a browser used for browsing the WEB page.

The adviser sends a kit (blood collecting tubes 1a, 1b), a blood-collecting needle, and a funnel to the user who has made application. Moreover, the adviser also sends a simple centrifugal blood separator to the user who has made application for the first time. The user drops blood into each of the blood collecting tubes 1a, 1b. The blood in the blood collecting tube 1a is centrifuged in order to separate the blood into a blood clot and serum. After that, the adviser receives the kit, the blood-collecting needle, and the funnel from the user, and then inspects blood components.

In addition, the user browses the WEB page of the server using the browser of the terminal equipment. The WEB page displays, for example, questions as well as a doctor in the form of a moving image or a still image to show a screen that enables the user to image actual doctor's interview. Alternatively, it may also be a screen displaying only question columns and answer columns. The server receives answers, which have been inputted into the WEB page, from the terminal equipment.

The morbid state analysis expert system receives input of inspection data relating to blood components. In addition to it, the morbid state analysis expert system receives the user's answers from the server. Steps after this, by which the expert system creates advice to report the advice to the user, are similar to those in the embodiment described above.

Additionally, in each of the embodiments described above, the adviser makes a report by sending an advice report to the user. In this manner, instead of sending the report printed by the morbid state analysis expert system 21 or other terminal equipment, a report may also be made by directly transmitting the created advice as electronic data to the user's terminal equipment via a communication line. For example, the advice may also be transmitted as electronic mail. Alternatively, the advice may also be transmitted as WEB page screen information in response to a request from the user's terminal equipment. A doctor may also be displayed on this WEB page in the form of a moving image or a still image to enable the user to imagine as if an actual doctor is giving advice.

Moreover, if inspection results relating to blood components are inputted into the morbid state analysis expert system 21 before the WEB page of the server is accessed by the user, it is possible to report advice immediately to the user who has accessed the WEB page. In this case, the user sends the kit, the blood-collecting needle, and the funnel in the first place. Then, the user accesses the WEB page after a lapse of time required for inspection. During the period of time, the adviser carries out the inspection, and then inputs inspection data into the morbid state analysis expert system 21. If the server, which has been accessed by the user, receives answers to doctor's questions, the server transmits the answers to the morbid state analysis expert system 21. The morbid state analysis expert system 21 creates advice immediately on the basis of inputted inspection data and the answers received from the server, and then transmits the advice to the server. The server presents the advice information to the user as a WEB page. Reporting the advice in this manner permits the user to receive the advice report immediately after inputting answers to the doctor's questions.

In each of the embodiments described above, a case where the kit has the blood collecting tubes 1a, 1b is shown. However, the kit may also have only either of the blood collecting tubes to give only advice on the basis of inspection of serum components, or to give only advice on the basis of inspection of blood components which are not hemolyzed.

### Industrial Applicability

According to the health advising method of the present invention, a medium for storing information about health is sent to a user, the medium in which the user stores information about health is received, and then the morbid state analysis expert system creates advice on the basis of the information about health stored in the medium. Therefore, the user can prepare the information about health at user's convenience. In addition, a person who gives advice can give appropriate advice to many people.

In addition, a health advice system according to the present invention is configured to comprise a morbid state analysis expert system that inputs information about health extracted from a medium for storing information about user's health, and that creates advice about user's health. Thus, if the user prepares a medium in which information about health is stored, the user can be advised, which eliminates the need for sparing time in order to be advised. Further, the person who gives advice can give appropriate advice to a lot of people.

## Claims

1. A health advising method comprising the steps of:
in response to application from a user, sending a medium for storing information about health to the user;
receiving the medium in which the user has stored information about health;
extracting the information about user's health stored in the medium to input said information in a morbid state analysis expert system;
creating advice about user's health by the morbid state analysis expert system; and
reporting the advice about user's health created by the morbid state analysis expert system to the user.

2. A health advising system comprising:
a morbid state analysis expert system that inputs information about health extracted from a medium for storing information about user's health, and that creates advice about user's health.

3. A health advising system according to Claim 2, wherein:
the medium for storing information about health comprises a question sheet about life in which questions are described, and in which answers to the questions are written by the user; a blood-collecting tool used for dropping blood of the user; and a kit for storing the dropped blood; and
the morbid state analysis expert system creates advice about user's health on the basis of the answers to the questions written in the question sheet about life, and on the basis of components of the blood stored in the kit.

4. A health advising system according to Claim 2, wherein:
the medium for storing information about health comprises a blood-collecting tool used for dropping blood of the user; and a kit for storing the dropped blood; and
the morbid state analysis expert system receives answers to given questions via a communication network, and creates advice about user's health on the basis of components of the blood stored in the kit and the answers.

5. A health advising system according to Claim 3 or 6, wherein:
the kit for storing blood has a separating agent for separating blood into a blood clot and serum, said kit being mounted on a centrifugal separator by a user, said kit storing the blood clot and the serum which are separated by the separating agent and by centrifuging the blood; and
the morbid state analysis expert system creates advice about user's health on the basis of components of the separated serum.

6. A health advising system according to Claim 3 or 4, wherein:
the kit for storing blood has a preservative for preventing hemolysis, and stores blood together with the preservative; and
the morbid state analysis expert system creates advice about user's health on the basis of components of the blood stored together with the preservative.

7. A health advising method according to Claim 1, wherein:
the medium for storing information about health comprises a blood-collecting tool used for dropping blood of the user; and a kit for storing the dropped blood; and
in a step in which, in response to application from a user, a medium for storing information about health is sent to the user, a centrifugal separator for separating the blood in the kit into a blood clot and serum is sent together with the medium to the user who makes application for the first time.
